# EUROPEAN PATENT APPLICATION

(11) **EP 0 992 792 A2**
(43) Date of publication of application: **12.04.2000**
(21) Application number: 99307891.4
(22) Date of filing: 07.10.1999
(51) Int. Cl.: G01N 33/28, G01N 1/34, G01N 1/38, G01N 1/40

(54) **Fluid analyte measurement system**

(30) Priority: 07.10.1998 US 168019
(71) Applicant: GENERAL ELECTRIC COMPANY, Schenectady, NY 12345 (US)
(72) Inventor: Shapiro, Andrew Philip, Schenectady, New York 12309 (US); Gui, John Yupeng, Niskayuna, New York 12309 (US); Pareek, Vinod Kumar, Niskayuna, New York 12309 (US); Whitehead, Alan, Charlton, New York 12019 (US)
(74) Representative: Szary, Anne Catherine, Dr.

(57) **Abstract**

A fluid analyte measurement system includes an extraction unit having a fluid flow path in intimate contact with an extraction solution flow path. The fluid flow path includes a fluid entry port and a fluid exit port and the extraction solution flow path includes an extraction solution flow entry port and an extraction solution flow exit port. The extraction unit extracts analytes from a fluid flow introduced within the fluid flow path through exposure of the fluid flow to an extraction solution introduced within the extraction solution flow path. A sample head vessel is coupled to the extraction solution flow exit, port and at least one probe is coupled to the sample head vessel to generate signals indicative of the characteristics of the fluid flow analytes within the extraction solution flow. For example, this measurement system can be used to determine Na' level within fuel oil. Circuitry is coupled to the probe(s), which circuitry is configured to measure the signals generated by the probe(s).

## Description

This application relates generally to fluid analyte measurement systems, and more specifically relates to a combustible fuel contaminant measurement system.

Industry currently utilizes several techniques for determining levels of constituents in products. For example, techniques for measuring sodium in organic liquids include atomic absorption, inductively coupled plasma (ICP) with atomic emission, mass spectrometry, and ion chromatography. The importance of detecting sodium level is exemplified in a current sodium contaminant problem in the manufacture of high temperature gas turbine components. The alloys utilized in these components are susceptible to hot corrosion, which corrosion is promoted by sodium concentration in fuel. The sodium found in distillate fuels usually originates during shipment of the fuel in ocean tankers or trucks.

One current problem with most measuring techniques is that these techniques are designed for laboratory conditions and are not suitable for most industrial applications. The vibrations and ambient environment at a power plant are not suitable for these analytical techniques and systems. In conventional settings, a sample of fuel is taken to a remote laboratory and analyzed.

Therefore, it is apparent from the above that there exists a need in the art for improvements in fluid analyte measurement systems, especially in combustible fuel contaminant measurement applications.

### SUMMARY OF THE INVENTION

A fluid analyte measurement system comprises an extraction unit having a fluid flow path in intimate contact with an extraction solution flow path. The fluid flow path comprises a fluid flow entry port and a fluid flow exit port and the extraction solution flow path comprises an extraction solution flow entry port and an extraction solution flow exit port. The extraction unit extracts analytes from a fluid flow introduced within the fluid flow path through exposure to an extraction solution introduced within the extraction solution flow path. A sample head vessel is coupled to the extraction solution flow exit port and at least one probe is coupled to the sample head vessel to generate signals indicative of the characteristics of the fluid flow analytes within the extraction solution flow. Circuitry is coupled to the probe(s), which circuitry is configured to measure the signals generated by the probe(s). For example, this measurement system can be used to determine Na⁺ level within fuel oil.

### BRIEF DESCRIPTION OF THE DRAWINGS

FIG. 1 is a schematic representation of a fluid analyte measurement system in accordance with one embodiment of the instant invention;
FIG. 2 is a schematic representation of a fluid analyte measurement system in accordance with another embodiment of the instant invention;
FIG. 3 is a schematic representation of a fluid analyte measurement system in accordance with another embodiment of the instant invention;
FIG. 4 is a schematic representation of a fluid analyte measurement system in accordance with another embodiment of the instant invention;
FIG. 5 is a schematic representation of a fluid analyte measurement system in accordance with another embodiment of the instant invention;
FIG. 6 is a schematic representation of a fluid analyte measurement system in accordance with another embodiment of the instant invention;
FIG. 7 is a graphical representation of an online Na⁺ analysis with a membrane extraction unit in accordance with another embodiment of the instant invention;
FIG. 8 is a graphical representation of the measurement of sodium concentration in fuel oil without a membrane extraction unit in accordance with one embodiment of the instant invention; and
FIG. 9 is a graphical representation of the measurement of sodium concentration in fuel oil with a membrane extraction unit in accordance with one embodiment of the instant invention.

### DETAILED DESCRIPTION OF THE INVENTION

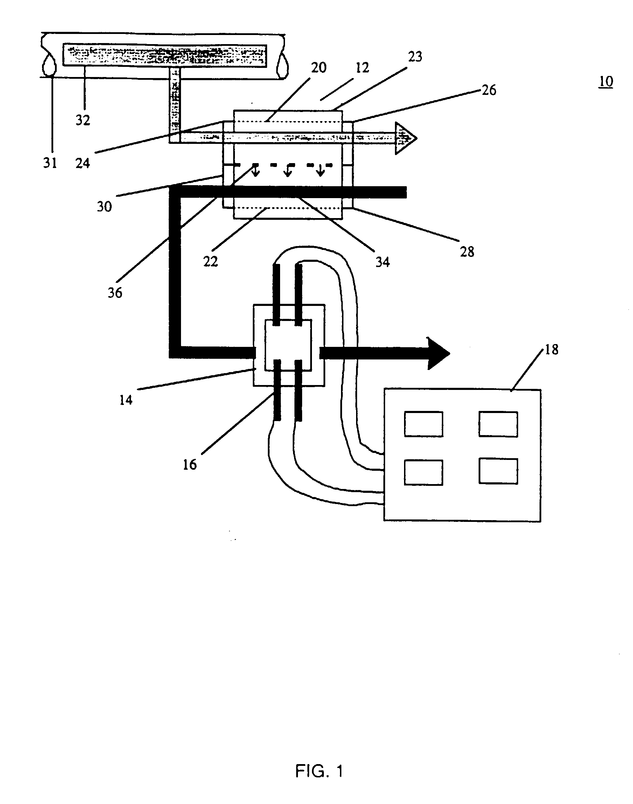

A fluid analyte measurement system 10 comprises an extraction unit 12, a sample head vessel 14, at least one probe 16 and circuitry 18 coupled to at least one probe 16, as shown in FIG 1.

Extraction unit 12 typically includes a fluid flow path 20 in intimate contact with an extraction solution flow path 22 disposed within a housing 23. Fluid flow path 20 and extraction solution flow path 22 are typically separated by a semi-permeable membrane 36, which membrane 36 supports the extraction of fluid analytes.

Fluid flow path 20 comprises a fluid flow entry port 24 and a fluid flow exit port 26 and extraction solution flow path 22 comprises an extraction solution flow entry port 28 and an extraction solution flow exit port 30. Extraction unit 12 extracts analytes from a fluid flow 32 introduced within fluid flow path 20 by intimately contacting fluid flow 32 with an extraction solution flow 34 introduced within extraction solution flow path 28. As used herein, the term "analyte" refers to a constituent part of a whole, for example, a contaminant or a specific element or compound found within a substance. Fluid flow 32, for example, may be extracted from a pipeline, or the like, by a slip stream process. Sample head vessel 14 is coupled to extraction solution flow exit port 30 by a conduit (not shown) and at least one probe 16 is coupled to sample head vessel 14 to generate signals indicative of the characteristics of fluid analytes in extraction solution flow 34. Circuitry 18 is coupled to at least one probe 16, which circuitry 18 is configured to measure the signals generated by at least one probe 16.

A desired increase in analyte concentration can be achieved in extraction solution flow 34 by controlling the ratio of flow rates of fluid flow 32 and extraction solution flow 34. Extraction solution flow 34 captures fluid analytes of fluid flow 32 through membrane 36 and analysis of fluid analytes is effected by directing extraction solution flow 34 into sample head vessel 14.

In one embodiment, extraction unit 12 is utilized for the extraction of salts in a combustible fuel. For example, when fluid flow 32, driven by a pressure gradient, intimately contacts membrane 36, which membrane 36 intimately contacts extraction solution flow 34, the sodium ions in fluid flow 32 are exchanged through membrane 36 and are conveyed to sample head vessel 14 by extraction solution flow 34. This process is achieved by diffusion of ions through membrane 36 to extraction solution flow 34. A pressure gradient is used to drive extraction solution flow 34 and enable conveyance of fluid analytes to sample head vessel 14. A problem of interference ions exists in the case of detecting and analyzing salts in fuel oil because ions such as lithium, silver, ammonium and potassium will interfere in the detection process. As a result, extraction solution flow 34 may be composed of deionized water and cherhicals,to adjust ionic strength and eliminate interfering ions from the solution. Such chemicals in extraction solution flow 34 may consist of, for example, pH buffers, ionic strengtheners, complexation agents or the like. PH buffers may comprise, for example, ammonium hydroxide, ammonia, borax, carbonates, ethanclamine, ethylamine and phosphates. Ionic strengtheners may comprise, for example, organic or inorganic salt that provides solution conductivity consisting of ammonium hydroxide, ammonium chloride, ammonium bromide, ammonium fluoride, calcium fluoride and calcium acetate. Complexation agents may comprise, for example, EDTA, 2,2dipyridyl, oxalate, and citric acid.

Membrane 36 may consist of, for example, cation exchange membranes, anion exchange membranes, hydrophilic membranes or the like. In addition, membrane material may consist of, but is not limited to polytetrafluoroethylene, polysulfone, cellulose acetate, cellulose nitrate, nylon, polystyrene divinylbenzene, Nafion® unreinforced and reinforced perfluorinated resin bearing sulfonic or carboxylic acid functionality. Membrane 36 may be configured, for example, as tubing, sheets, bags or the like.

Fluid flow 32 may contain analytes including but not limited to ammonia, ammonium, cadmium, calcium, carbon dioxide, chloride, chlorine, cupric, cyanide, fluoride, fluoroborate, iodide, lead, nitrate, nitrite, nitrogen oxide, oxygen, perchlorate, potassium, silver/sulfide, sodium and thiocyanate.

Fluid flow 32 may consist of organic liquids, for example, fuel oil, No.1 oil, No. 2 oil, JP-4 fuel oil, crude oil and the like. In addition, fluid flow 32 may be a gas, for example natural gas or air, which gas may contain analytes such as, but not limited to hydrochloric acid, nitric acid, hydrofluoric acid, nitrogen oxide, carbon dioxide, ammonia and the like. For example, fluid flow 32 may be a solution of gas in a liquid which gas may consist of analytes such as, but not limited to: hydrogen, ammonia, bromide, carbon dioxide, chloride, chlorine, fluoride, iodine, nitrogen oxide, sulfur oxide, oxygen, carbon monoxide, methanol, methane, hydroquinone, catechol and the like.

Sample head vessel 14 houses at least one probe 16, which probe(s) 16 typically comprise ion-selective electrode(s) that measure fluid flow 32 analytes, which electrode(s) may be used in analyte measurement methods including, but not limited to, potentiometry, voltammetry, coulometry and the like, as discussed in greater detail below. Although potentiometry, voltammetry and coulometry are mentioned, it is noted that any other analyte measurement methods could alternatively be utilized without deviating from the scope of the present invention. At least one probe 16 may further comprise a pH electrode, a temperature probe or an electrochemical cell for chloride reduction.

For example, the types of ion selective electrodes that may be used to measure analytes extracted from fluid flow 32 may include, but are not limited to, the following: ammonia, ammonium, cadmium, calcium, carbon dioxide, chloride, chlorine, cupric, cyanide, fluoride, fluoroborate, iodide, lead, nitrate, nitrite, nitrogen oxide, oxygen, perchlorate, potassium, silver/sulfide, sodium, thiocyanate probes and the like. While only the previous ion-selective electrodes are mentioned for a respective analyte measuring system, any number of ion selective electrodes may be utilized, and are within the scope of this invention. In one embodiment, electrodes in sample head vessel 14 measure ion concentration of extraction solution flow 34. In measuring the level of sodium in a fuel sample, a chloride ion selective electrode may be used in the case where sodium exists mainly as salt (NaCI). The chloride can be measured by potentiometry with a chloride ion selective electrode. The chloride, however, can also be measured by voltammetry and coulometry through electrochemical oxidation.

Potentiometry is an electrochemical method that measures potential of a specially designed ion selective electrode. The electrode potential changes as a function of the ion concentration. Potentiometry is a simple electrochemical method, but its speciation ability to distinguish similar ions is limited. For example, a Na⁺ ion selective electrode is considered highly selective to Na⁺ when comparing its response to ions that are not similar to Na⁺ such as: anions and double charged cations. However, Na⁺ family ions such as Li⁺, K⁺, Rb⁺ and Na⁺ similar ions such as Ag⁺, Tl⁺ and NH4⁺, cause certain levels of interference to Na⁺ measurement. Among these interference ions, Ag and Li ions can cause significant measurement error for Na⁺ because Na⁺ ion selective electrodes have similar or even greater response factor to these ions. Thus, it is desirable to measure Na⁺ concentration in the absence of these strong interference ions. For the weak interference ions such as Rb⁺ and NH₄⁺, it is not as important to eliminate their presence as it is to keep their concentration constant.

Voltammetry measures electrode current as a function of electrode potential. The electrode current results from the oxidation or reduction of the analyte(s) at the electrode surface, thus proportional to the analyte concentration in solution. Since many chemicals have a unique oxidation or reduction potential, voltammetry can provide speciation through its potential scan. In this way, different analytes can be oxidized or reduced at different potentials. However, voltammetry cannot be applied directly to electrochemically inactive species, which species cannot be oxidized or reduced at the electrode within the specified environment. For example, Na⁺ cannot be oxidized or reduced in aqueous solution, thus it cannot be measured by voltammetry. But Na⁺ may be indirectly measured by voltammetry through Cl⁻ oxidation if Na⁺ contamination in oil comes conclusively form salt (NaCI). The Cl⁻ oxidation current provides direct measurement for Cl⁻ and indirect for Na⁺. It should be mentioned that Cl⁻ can also be measured by potentiometry through its ion selective electrode, but the interference from other similar ions such as I and Br can produce significant measurement error as to measure Cl⁻ through voltammetry. Furthermore, if Cl⁻ voltammetry and Na⁺ potentiometry can be run in parallel, Na⁺ measurement reliability can be significantly improved. For example, if both methods produce the same result within a specified tolerance, the measurements are most likely accurate. If they yield different results, one of the measurements may be wrong and the result should be studied.

Coulometry measures current and charge (current ∗ time) from an electrode as a function of time in a potential step experiment. For example, one can measure Cl⁻ concentration by monitoring oxidation current or charge in the experiment where the electrode potential is stepped from a rest potential (at which Cl⁻ cannot be oxidized) to an oxidation potential (where Cl⁻ will be oxidized).

Circuitry 18 is coupled to at least one probe 16, which circuitry 18 is configured to measure the signals generated by at least one probe 16. In one embodiment of the instant invention, circuitry 18 is a meter having input ports (not shown) for probes 16 (electrodes). Circuitry 18 will provide output as to the level of analyte in the fluid flow 32. In one embodiment, the output from the circuitry 18 can be used for process control.

A temperature probe, for example a thermocouple, thermistor or the like, may be helpful in electrochemical measurement because of the sensitivity of electrochemical equilibria and rates on temperature. In addition, a pH electrode will indicate the acidity of the solution and will provide an indication of a gradual drift of a fixed pH extraction solution. For example, Na⁺ ion selective electrode is responsive to H⁺ ions, thus its potential is a function of solution pH. The solution pH has to be maintained above 9 when measuring 10⁻⁴M Na⁺ to avoid considerable false positive measurements. Thus, for measuring Na⁺ concentration in a process stream where pH value is not constant, it is thus necessary to monitor H⁺ concentration so that its contribution to the electrode potential can be deducted.

In the operation of an exemplary fluid analyte measuring system 10, a slipsteam of fluid flow 32, typically a combustible fuel or the like, is extracted from a pipeline 31 into extraction unit 12. In extraction unit 12, fluid flow 32 and extraction solution flow 34, typically an aqueous solution, are each disposed in intimate contact with semi-permeable membrane 36 to promote ion exchange therebetween. As extraction solution 34 contacts membrane 36, analytes within fluid flow 32 are exchanged with extraction solution 34 through membrane 36. Extraction solution 34 containing certain selected analytes from fluid flow 32 is pumped to sample head vessel 14. At least one probe 16 is disposed within sample head vessel 14 to generate signals indicative of the level of analytes present within extraction solution 34. Circuitry 18 is electrically coupled to at least one probe 16.

Many different signature characteristics of fluid flow 32 can be captured by such a system. At least one probe 16 is selected based on the signature characteristics sought. For example, if a system user wants to detect the level of a contaminant, such as sodium, within fuel oil, an ion selective probe may be utilized. Circuitry 18, is typically configured as a meter or the like connected to at least one probe 16 to generate indicia indicative of analyte level such that a system user can read or verify the analyte level and take appropriate action, such as a system shutdown.

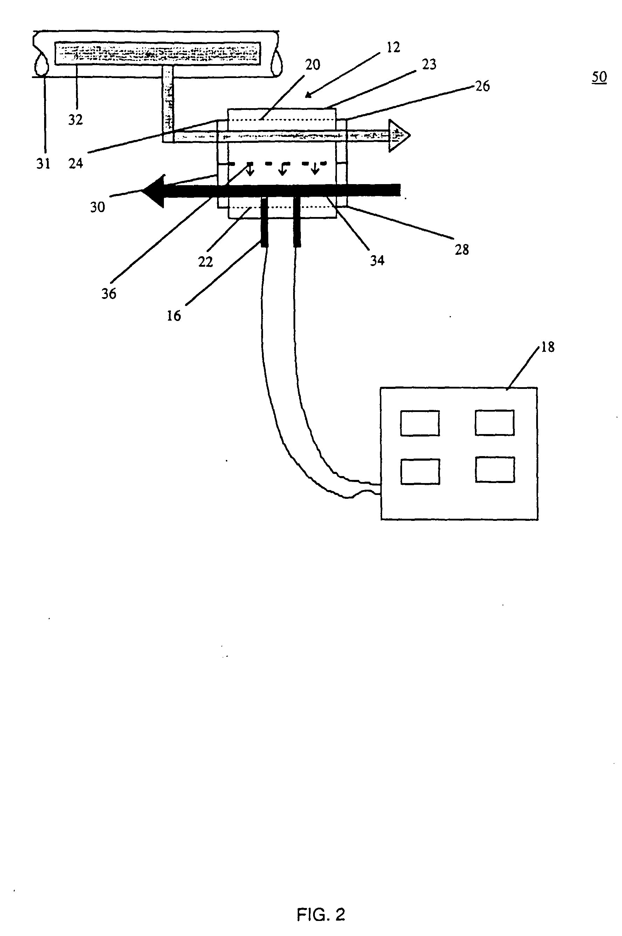

An alternative embodiment 50 of the instant invention is shown in FIG 2. The embodiment is similar in all respects to fluid analyte measurement system 10 of FIG 1, except that sample head vessel is not utilized and at least one probe 16 is directly coupled to an extraction unit 12 within extraction solution flow path 22 and probe(s) 16 are in intimate contact with extraction solution flow path 22. In another embodiment of the instant invention, extraction solution flow 34 as shown in FIG. 2 may be stepwise, rather than continuous.

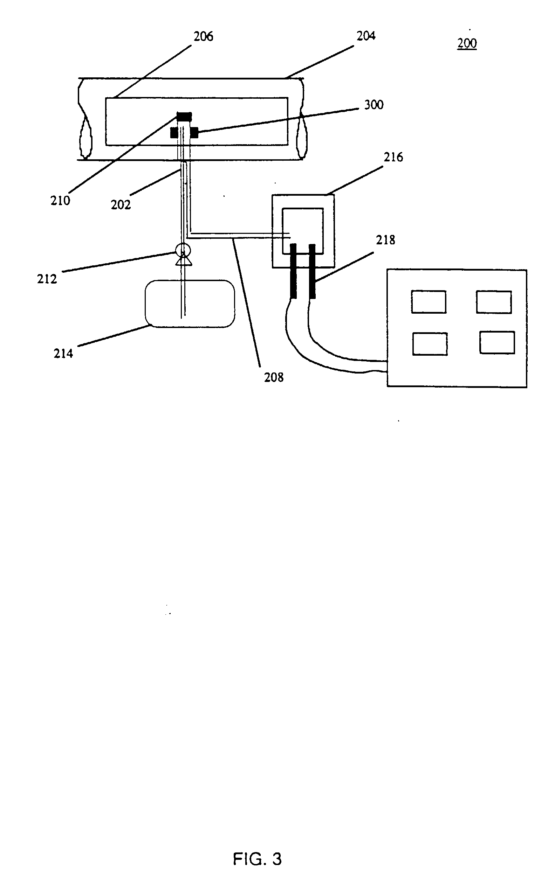

Another embodiment 200 of the instant invention is shown in FIG 3. An in-line membrane filtration probe 202 is disposed within a pipeline 204 for detecting analytes within a fluid flow 206 therein. In-line probe 202 comprises an extraction solution flow path 208 and a membrane 210, which membrane 210 is in intimate contact with fluid flow 206. A pump 212 pumps an extraction solution 214 through extraction solution flow path 208. As extraction solution 214 passes membrane 210, analytes within fluid flow 206 are exchanged with extraction solution 214 through membrane 210. Extraction solution 214 containing certain selected analytes from fluid flow 206 is then pumped to a sample head vessel 216. At least one probe 218 is disposed within sample head vessel 216 to generate signals indicative of the level of analytes present within extraction solution 214. Circuitry 220 is electrically coupled to at least one probe 218.

Many different signature characteristics of fluid flow 206 can be captured by such a system. Probe 218 is selected based on the signature characteristics sought. For example, if a system user wants to detect the level of contaminants such as sodium within fuel oil, an ion-selective probe may be utilized. Circuitry 220, is typically configured as a meter or the like connected to at least one probe to generate indicia indicative of analyte level such that a system user can read or verify the analyte level and take appropriate action, such as a system shutdown. Examples of various fluid flows 206, analytes, membrane 210 types and probes 218 are discussed in greater detail above.

In-line membrane filtration probe 202 may further comprise at least one sonication ring 300 disposed about membrane 210. Fuel oil may contain pockets of water that store Na⁺ analytes. In order to get a representative sample of Na⁺, sonication ring 300 may be used to vibrate and disperse water imbedded in fuel oil.

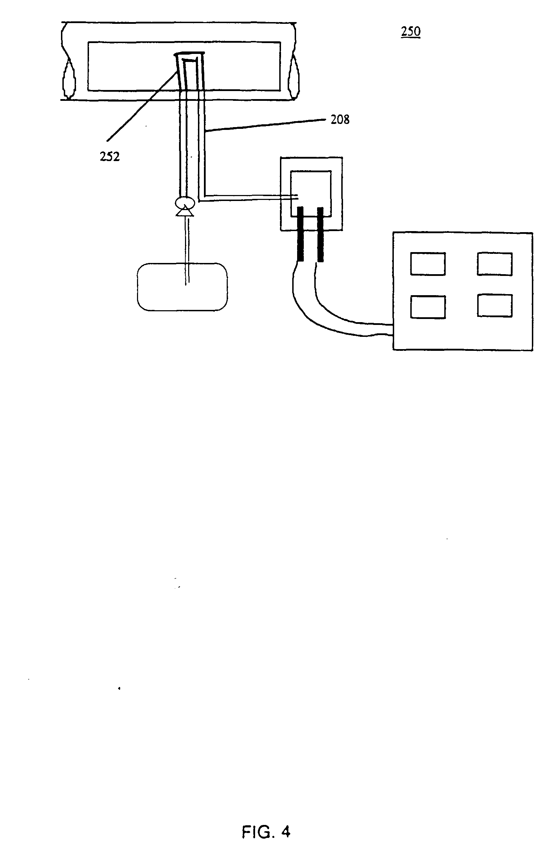

Another embodiment 250 of the instant invention is shown in FIG 4. Embodiment 250 is similar in all respects to embodiment 200 of FIG 3, except that membrane 210 of FIG 3 is replaced by a membrane tubing 252 disposed within extraction solution flow path 208.

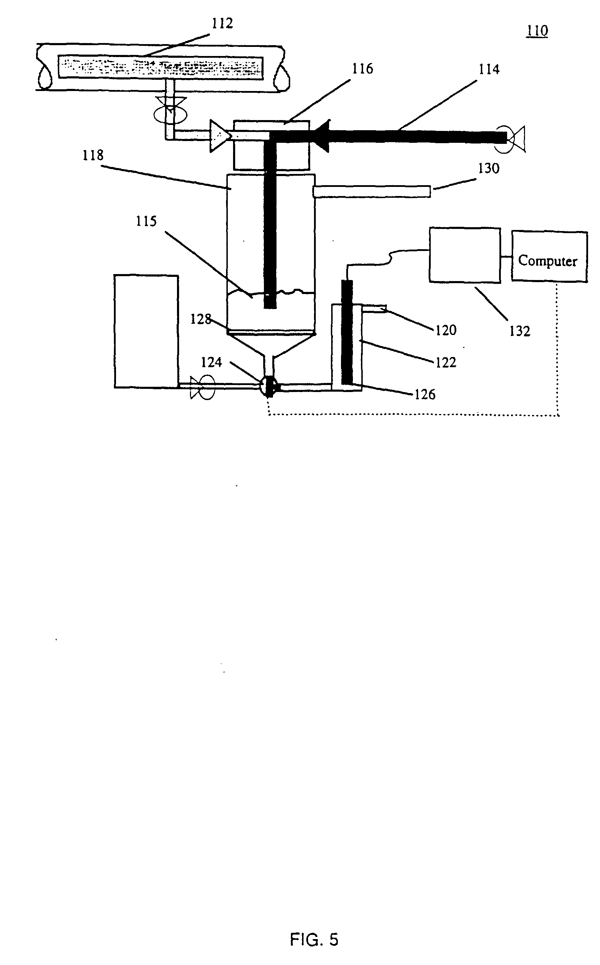

In an alternative embodiment 110 as shown in FIG 5, an extraction apparatus for online analyte analysis is shown. A fluid flow 112 and an extraction solution flow 114 are pumped into a static mixer 116 and mixed. The mixing action serves as an extraction process to transfer analytes from fluid flow 112 into extraction solution flow 114. For example, fuel oil contains pockets of water which house Na⁺ analytes. The mixing action disperses water imbedded in fuel oil, which water mixes with extraction solution flow 114. The resulting mixture of fluid flow 112 and extraction solution flow 114 flows into extraction vessel 118, which extraction vessel 118 is pre-filled with a quiescent extraction solution 115 to a level of an exit tubing 120 in sample head vessel 122.

For example, in the measurement of Na⁺ in fuel oil, the water droplets (which contain Na⁺ analytes) will aggregate into larger water droplets by use of static mixer 116. The contents in static mixer 116 are then transferred to quiescent extraction solution 115 in extraction vessel 118. Quiescent extraction solution 115 typically comprises the same fluid as extraction solution flow 114. Fuel oil will rise to the surface of both extraction solution flow 114 and quiescent extraction solution flow 115 (assuming both solutions are the same) since fuel oil density is less than the density of water. Fuel oil then exits the extraction vessel 118 from the top exit opening 130. Extraction solution flow 114 with Na⁺ is left behind, mixed with quiescent extraction solution 115. This mixture will cause the increase of the solution level, providing a driving force for the flow into sample head vessel 122.

Sample head vessel 122 is coupled to extraction vessel 118 with a valve 124 disposed therebetween. At least one probe 126 electrically coupled to circuitry 132 is disposed in sample head vessel 122. At least one probe 126 and circuitry 132 perform as discussed above.

If a continuous measurement of analyte level is desired, valve 124 is placed in open position to allow the mixture of extraction solution flow 114 and quiescent extraction solution 115 with analyte to flow into sample head vessel 122 and at least one probe 126, typically an ion selective electrode, continuously measures the concentration of analyte in the mixture. For example, Na⁺ may be continuously measured by a Na⁺ ion selective electrode.

Additionally, an oil filter 128 may be disposed at the bottom of extraction vessel 118 to remove remaining oil residue in the mixture of extraction solution flow 114 and quiescent extraction solution 115. Similarly, an oil removal cartridge may be used to remove residue.

If a batch mode measurement of analyte level is desired, valve 124 at extraction vessel 118 is placed in an open position at preset time intervals. In a batch mode measurement, it is not necessary to mix extraction solution flow 114 with fuel oil sample before entering extraction vessel 118. For example, fuel oil may be pumped directly into extraction vessel 118 and any water droplets (where Na⁺ resides) in fuel oil will be left with quiescent extraction solution 115. Although the extraction of analytes may not be as complete as using a static mixer 116 as described above, Na⁺ concentration is significantly enriched in the quiescent extraction solution 115. The enrichment factor is determined roughly by volume ratio of fuel oil over the extraction solution flow 114 in extraction vessel 118. For example, if extraction vessel 118 is filled with 10ml of quiescent extraction solution 115 and 400ml oil sample is pumped into extraction vessel 118, the enrichment factor is 40. However, in continuous measurement of analyte mode, enrichment factor is determined by the flow ratio of oil and extraction solution. For online instruments, it is desired to have a small sample flow because handling large flow adds cost, space, and waste. In addition, it is difficult for an online operation to maintain constant flow ratio compared with volume ratio. In summary, batch mode measurement of analytes provides high detection sensitivity because of higher Na⁺ enrichment, and continuous measurement of analytes provides faster response through continuous real time measurement.

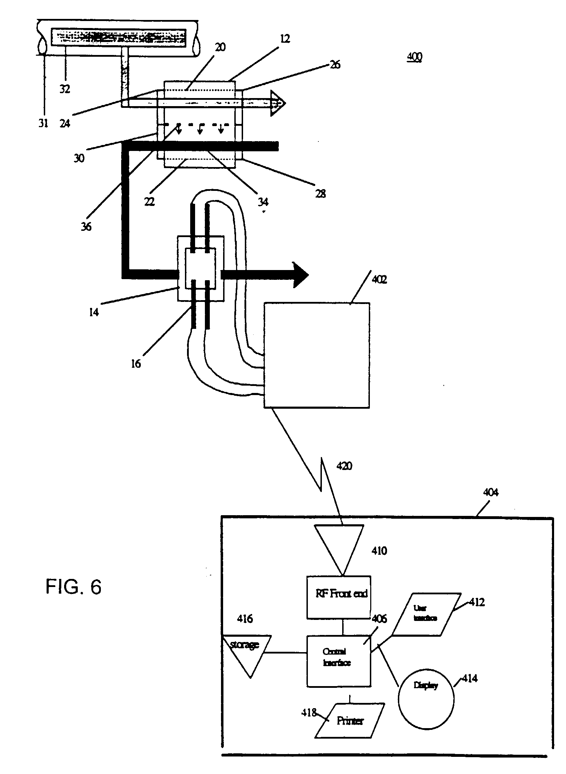

A remote fluid analyte measurement system 400 is shown in Fig. 6. This embodiment is similar in all respects to fluid analyte measurement system 10 of figure 1, except that at least one probe 16 is electronically coupled to at least one remote unit 402 for transmitting signals from fluid analyte measuring system 400.

A remote station 404 provides a communication base for interaction with a respective remote unit 402. Remote station 404 typically comprises a central interface 406, a radio frequency (RF) front-end 408, an antenna 410 and user interface related peripheral devices including a user interface 412, a display 414, data storage 416 and a printer 418 for enabling a user to input relevant information into central interface 406. Peripheral devices as defined in this application include any device for storing analyte measurement or analysis information and intelligibly communicating the same to a system user, and include such devices as printers, hard disk drives, floppy disk drives, cathode ray tubes (CRTs) and keyboards. While only one set of respective peripheral devices are shown for a respective central interface 406, any number of peripheral devices may be utilized and are within the scope of the instant invention.

Communication between remote station 404 and a respective remote unit 402 is by way of a communication system 420, such as a "geo-synchronous" "L-band" satellite system, a " Little Leo" satellite system, a two-way paging system, a modem connection or any communication system capable of two-way communication between remote station 404 and a respective remote unit 402.

### EXAMPLE

A batch mode measurement of analyte level experiment was performed using three oil samples prepared by adding 0.5% water containing 0, 210, 1050 ppm Na⁺ into about 420 ml No. 2 distillate fuel oil. The actual Na⁺ concentration in these three oil samples was 0, 1.25 and 6.18 ppm. The sample solutions were stirred so that the oil and water were mixed during the experiment. The sample was pumped into extraction vessel 118 that was pre-filled with 10 ml of quiescent extraction solution 115 (0.036 M NH₄CI and 0.036 M NH₄OH). The pump rate was set at 4.6ml/min and stopped after 40 ml oil sample was obtained. The extraction solution was then transferred into sample head vessel 122 for Na⁺ determination. The experiment was then continued for subsequent oil samples. The final results were as follows:

| Oil sample containing Na⁺ (ppm) | Potential Measured (mV) |
|---|---|
| 0.00 | -260 |
| 1.25 | -172 |
| 6.18 | -150 |

### EXAMPLE

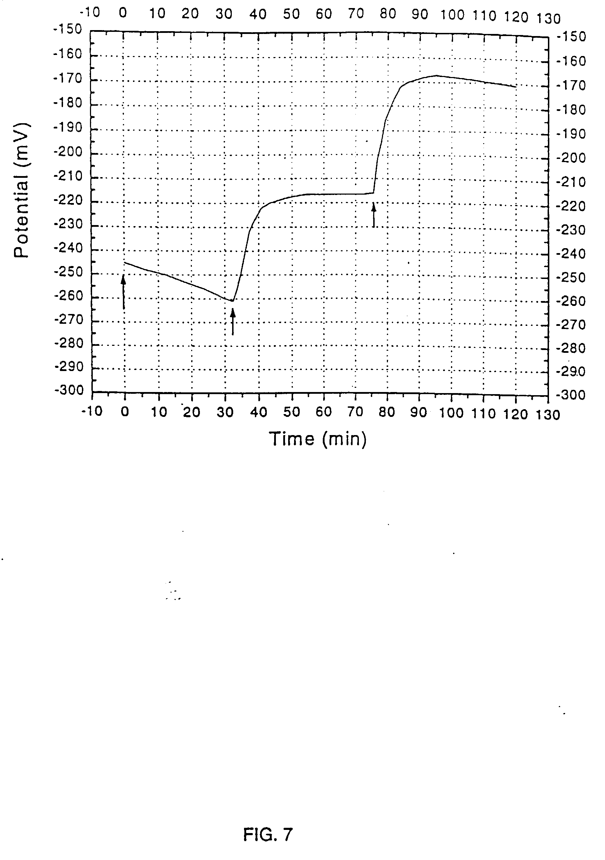

FIG 7 depicts a graphical representation of an online Na⁺ analysis with an extraction unit 12 utilizing a membrane 36 (see FIG. 1 for schematic). In one embodiment, Nafion® 450 is the material used for membrane 36 for the extraction of Na⁺ analytes.

A continuous mode experiment was performed using three oil samples prepared by adding 0.5% water containing 0, 210, 1050 ppm Na⁺ into about 420 ml No. 2 distillate fuel oil. The actual Na⁺ concentration in these three oil samples was 0, 1.25 and 6.18 ppm. The sample solutions were stirred so that the oil and water were well mixed during the experiment. The sample was pumped into fluid flow path 20 at 4.9 ml/min and extraction solution flow 34 (0.036M NH₄Cl and 0.036M NH₄OH) was pumped into extraction solution flow path 22 at 0.63 ml/min. Extraction solution flow 34 was continuously fed into the sample head vessel 14, which sample head vessel 14 comprises a dead volume of 1.0 ml. The results were as follows:

| Oil sample containing Na⁺ (ppm) | Potential Measured (mV) |
|---|---|
| 0 | gradual drift |
| 1.25 | -216.5 |
| 6.18 | -167 |

The gradual drift for 0 ppm Na⁺ samples was likely caused by residual Na⁺ in a Nafion® membrane that was not completely removed. The measured potential increased as 1.25 ppm Na⁺ sample was pumped into extraction and reached a steady state level after 20 minutes. The plateau potential was -216.5mV, corresponding to 0.609 ppm Na⁺ in extraction solution. The 0.609 ppm was part of the original 1.25 ppm Na⁺ sample that actually filtered through the membrane. Similarly, plateau potential for 6.18 ppm Na⁺ sample was -167mV, corresponding to 2.09 ppm Na⁺ in extraction solution. The 2.09 ppm was part of original 6.18 ppm Na⁺ sample that actually filtered through the membrane. The ratio of Na⁺ in the oil sample and in extraction solution is 0.495 for the present experiment (2.09 ppm/6.18 ppm = 0.495).

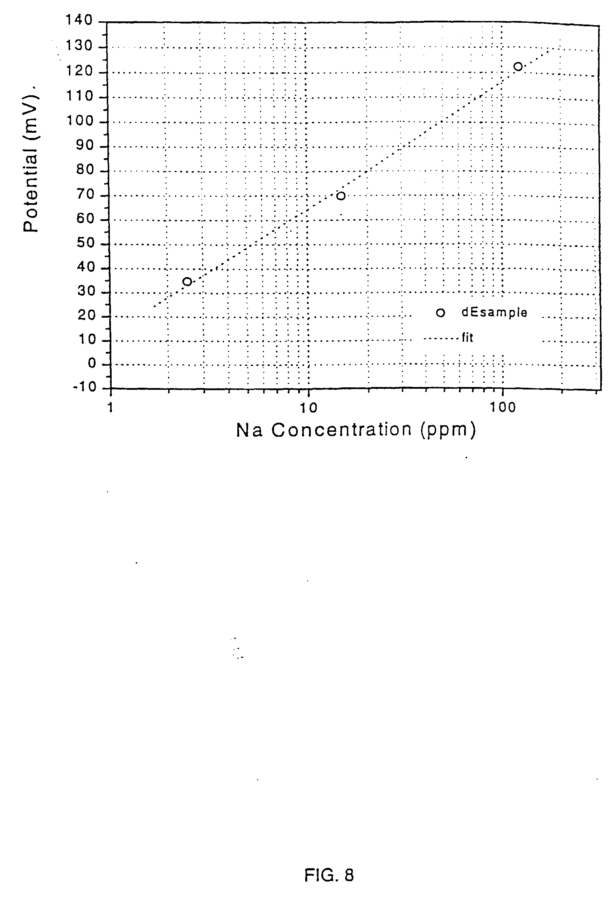

FIG 8 is a graphical measurement of sodium concentration in fuel oil with a liquid/liquid extraction. Four oil samples were made by adding 0, 0.048, 0.267, and 2.32 ml of synthesized sea water (with 10500 ppm Na⁺ as NaCI) into four containers with 200 ml of distillate fuel resulting in sodium concentrations of 0, 2.5, 14.7, and 122 ppm respectively. The oil sample and aqueous buffer solution (0.36M NH₄Cl and 0.36M NH₄OH) were placed into 50ml plastic test tubes and into a ultrasonication bath for 30 minutes. After the elapsed time, the samples gradually separated into oil and aqueous phases. The oil component was discarded and potential measurement was performed with a Na⁺ ion selective electrode on the remaining aqueous solution. The results as displayed in FIG 8 represent an average of three measurements.

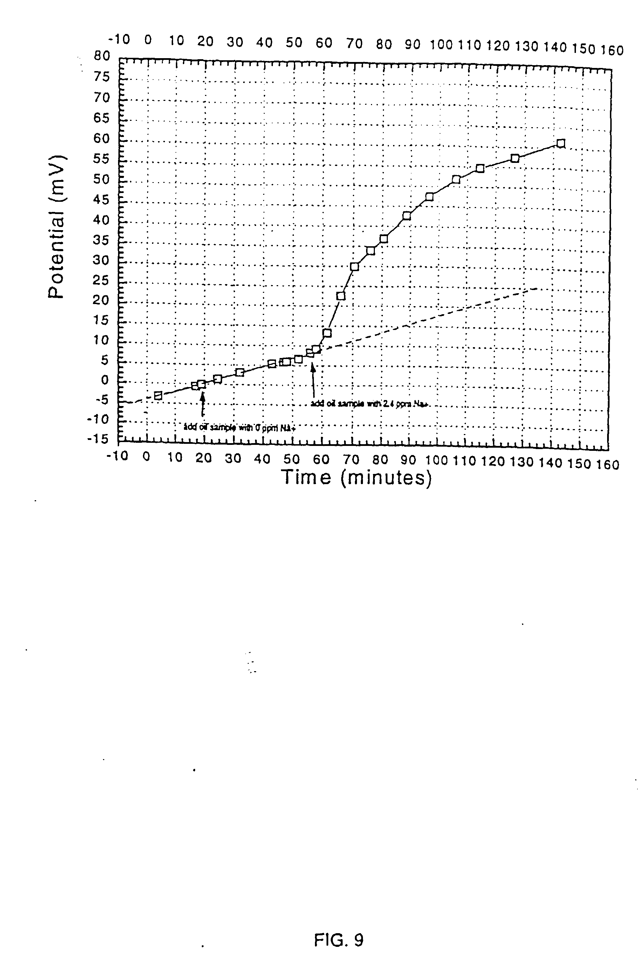

FIG 9 is a graphical measurement of sodium concentration in fuel oil with a membrane extraction. The membrane extraction unit (figure not shown) has two compartments, an extraction solution compartment and an oil compartment, separated by a cation exchange membrane (Nafion® 45) with a teflon coated stirrer at the bottom of each compartment. 35 ml of extraction solution (0.36M NH₄Cl and 0.36M NH₄OH) was placed in the extraction solution compartment. The Na⁺ ion selective electrode was placed inside the extraction solution in the extraction solution compartment and a potential reading was taken continuously. The experimental result shows that there was a potential drift from 0 to 19 minutes on the time scale when 75 ml of a blank oil sample (no sodium) was added into the oil compartment. After the oil addition, measurements revealed no noticeable potential change except for the drift for more than 20 minutes. The sample was taken out of the oil compartment and 75 ml of new oil sample containing 2.5 ppm Na⁺ and about 1% water was added into the oil compartment. As shown graphically in FIG 9, potential increases as a result of the Na⁺ ion diffusion into buffer solution through membrane 36. As time increases, the diffusion rate decreases as a result of the Na⁺ ion concentration depletion in oil phase and results in a gradual leveling of the ion selective electrode potential.

While the invention has been described with reference to a preferred embodiment, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims.

## Claims

1. A fluid analyte measurement system (10) coupled to a fluid flow (32) comprising:
an extraction unit (12) having a fluid flow path (20) in intimate contact with an extraction solution flow path (22) wherein said fluid flow path (20) comprises a fluid entry port (24) and a fluid exit port (26) and said extraction solution flow path (22) comprises an extraction solution entry port (28) and an extraction solution exit port (30) wherein said extraction unit (12) extracts analytes from a fluid flow (32) introduced within said fluid flow path (20) with an extraction solution flow (34) introduced within said extraction solution flow path (22);
a sample head vessel (14) coupled to said extraction solution exit port (30);
at least one probe (16) coupled to said sample head vessel (14) to generate signals indicative of analyte level within said extraction solution flow (34); and
circuitry (18) coupled to said probe (16), which circuitry (18) is configured to measure said signals.

2. A fluid analyte measurement system (10) in accordance with claim 1, wherein said fluid flow (32) comprises a combustible fuel.

3. A fluid analyte measurement system (10) in accordance with claim 2, wherein said combustible fuel is a fuel selected from the group consisting of No.1 oil, No. 2 oil, JP-4 fuel oil, and crude oil.

4. A fluid analyte measurement system (10) in accordance with claim 2, wherein said combustible fuel contains analytes selected from the group consisting of ammonia, ammonium, cadmium, calcium, carbon dioxide, chloride, chlorine, cupric, cyanide, fluoride, fluoroborate, iodide, lead, nitrate, nitrite, nitrogen oxide, oxygen, perchlorate, potassium, silver sulfide, sodium and thiocyanate.

5. A fluid analyte measurement system (10) in accordance with claim 1, wherein said fluid flow (32) is a gas.

6. A remote fluid analyte measurement system (400) coupled to a fluid flow (32) for conducting analyte level assess ment on at least one remotely located fluid analyte measuring system comprising:
an extraction unit (12) having a fluid flow path (20) in intimate contact with an extraction solution flow path (22) wherein said fluid flow path (20) comprises a fluid entry port (24) and a fluid exit port (26) and said extraction solution flow path (22) comprises an extraction solution entry port (28) and an extraction solution exit port (30) wherein said extraction unit (12) extracts analytes from a fluid flow (37) introduced within said fluid flow path (20) with an extraction solution flow (34) introduced within said extraction solution flow path (22);
a sample head vessel (14) coupled to said extraction solution exit port (30);
at least one probe (16) coupled to said sample head vessel (14) to generate signals indicative of analyte level within said extraction solution flow (34);
at least one remote unit (402) for transmitting said signals from said fluid analyte measuring system (400);
a central station (404); and
a communications link (420).

7. A combustible fuel contaminant measurement system (10) coupled to a combustible fuel source (32) comprising:
an extraction unit (12) having combustible flow path (20) in intimate contact with an aqueous solution flow path (22) wherein combustible fuel flow path (20) comprises a combustible fuel flow entry port (24) and a combustible fuel flow exit port (26) and said aqueous solution flow path (22) comprises an aqueous solution flow entry port (28) and an aqueous solution exit port (30) wherein said extraction unit (12) extracts contaminants from. a combustible fuel flow (32) introduced within said combustible fuelflow path (20) with an aqueous solution flow (34) introduced within said aqueous solution flow path (22);
a sample head vessel (14) coupled to said aqueous solution exit port (30);
at least one probe (16) coupled to said sample head vessel (14) to generate signals indicative of contaminant level within said aqueous solution flow (34); and
a meter (18) coupled to said probe (16), which meter (18) is configured to measure said signals.

8. A fluid analyte measurement system coupled to a fluid flow comprising:
an extraction unit (12) having a fluid flow path (20) in intimate contact with an extraction solution flow path (22) wherein said fluid flow path (20) comprises a fluid flow entry port (24) and a fluid flow exit port (26) and said extraction solution flow path (20) comprises an extraction solution flow entry port (28) and an extraction solution flow exit port (30) wherein said extraction unit (12) extracts analytes from a fluid flow (32) introduced within said fluid flow path (20) with an extraction solution flow (34) introduced within said extraction solution flow path (22);
at least one probe (16) coupled to said extraction unit (12) to generate signals indicative of characteristics of said analytes in said extraction solution flow path (22), and
circuitry (18) coupled to said probe (16) which circuitry (18) is configured to measure said signals.

9. A method of fluid analyte measurement comprising the steps of: introducing a fluid flow (32) into an extraction unit (12);
extracting fluid flow analytes from said fluid flow (32) through a semi-permeable membrane in said extraction unit (12) with an extraction solution flow (34);
generating signals indicative of characteristics of said analytes; and
measuring said signals to provide data regarding analyte levels and characteristics in said fluid flow.

10. An in-line membrane filtration probe (202) for direct readings within a pipeline (204) comprising:
an in-line membrane filtration probe (202) having an extraction solution flow path (208) in intimate contact with a fluid flow path wherein said in-line membrane filtration probe (202) extracts analytes from a fluid flow within said fluid flow path with an extraction solution flow introduced within said extraction solution flow path (208);
a sample head vessel coupled to said extraction solution flow path;
at least one probe (218) coupled to said sample head vessel to generate signals indicative of analyte level within said extraction solution flow; and
circuitry (220) coupled to said probe (218), which said circuitry (220) is configured to measure said signals.
